# EUROPÄISCHE PATENTSCHRIFT

(11) **EP 0 581 934 B1**
(45) Veröffentlichungstag und Bekanntmachung des Hinweises auf die Patenterteilung: **02.05.1997**
(21) Anmeldenummer: 93903954.1
(22) Anmeldetag: 11.02.1993
(51) Int. Cl.: A61F 2/80

(54) **OBERSCHENKELPROTHESE**
THIGH PROSTHESIS
PROTHESE FEMORALE

(30) Priorität: 14.02.1992 DE 4204482
(43) Veröffentlichungstag der Anmeldung: 09.02.1994
(73) Patentinhaber: POHLIG, Kurt, 83278 Traunstein (DE)
(72) Erfinder: POHLIG, Kurt, 83278 Traunstein (DE)
(74) Vertreter: Bauer, Robert, Dipl.-Ing.
(86) Internationale Anmeldenummer: EP9300332
(87) Internationale Veröffentlichungsnummer: WO9315695

(56) Entgegenhaltungen:
- EP-A- 0 019 612
- EP-A- 0 151 834
- DE-A- 2 540 138
- FR-A- 1 548 465
- US-A- 1 893 853
- US-A- 2 500 622
- US-A- 3 671 980
- US-A- 4 923 475
- US-A- 5 108 456
- US-A- 5 139 523

## Beschreibung

Die Erfindung betrifft eine Oberschenkelprothese gemäß Gattungsbegriff des Anspruchs 1.

Bei derartigen Prothesen ist man im allgemeinen bestrebt, den Schaft möglichst genau den Konturen des Gliedmaßenstumpfes anzupassen, um neben einem festen Sitz der Prothese eine möglichst großflächige Druckverteilung zu erreichen. Die Anpassung geht dabei neuerdings so weit, daß sich in dem Schaft infolge "Pumpens" beim Gehvorgang bewußt ein Unterdruck einstellt, wodurch der Schaft, ggf. sogar ohne zusätzliche Hilfsmittel, am Stumpf gehalten wird. Untersuchungen haben ergeben, daß, einen solchermaßen gut angepaßten Prothesenschaft vorausgesetzt, in dem Stumpf zu einem gewissen Grad ein hydrostatischer Druckausgleich stattfindet. Andererseits aber hat man auch gefunden, daß nicht alle Zonen des Stumpfes gleichermaßen für die Druckaufnahme geeignet sind, vielmehr prinzipiell neben druckempfindlicheren Zonen solche Zonen auftreten, die für die Aufnahme eines äußeren Druckes besser geeignet sind. Sodann hat man festgestellt, daß Volumenänderungen des Stumpfes, wie sie kurzfristig etwa durch Temperaturunterschiede und stoffwechselbedingte, u.a. tageszeitabhängige An- oder Abschwellungen, längerfristig durch Gewichtszu- oder -abnahme des Patienten, Muskelschwund oder dergl. auftreten, sich in gewissen Zonen stärker als in anderen bemerkbar machen. Während man der unterschiedlichen Druckaufnahmefähigkeit der verschiedenen Stumpfbereiche durch eine angepaßte, nicht blindlings konturabhängige Formgebung des Schaftes - im allgemeinen eines eigenen, in den äußeren Schaft herausnehmbar eingesetzten Innenschaftes - Rechnung zu tragen suchte, hat die letztgenannte Erscheinung zu Kompromissen gezwungen. Schließlich erschweren enge Schäfte aber auch das Anlegen ("Anziehen" oder "Adaptieren") der Prothese. Dabei wird zumeist so vorgegangen, daß über den Stumpf ein Strumpf gezogen wird, dessen loses Ende durch eine verschließbare bodennahe Öffnung des Schaftes hindurch nach außen gezogen wird. Sodann wird der Stumpf durch Zug an dem herausgezogenen Strumpfende in den Schaft hinein- und weiter der Strumpf durch die Öffnung herausgezogen, bis schließlich nur der Stumpf in dem Schaft zurückbleibt. Dieser Vorgang ist verständlicherweise bei einem engen Schaft schwierig.

Etwa aus den US-PSen 1 893 853 und 2 634 424 sowie dem DE-GM 1 810 432 - wovon im Gattungsbegriff ausgegangen wird - ist es bereits bekannt, um dem Prothesenschaft ungeachtet der sich ändernden Stumpfkontur eine gute Paßform und dem Prothesenträger ein angenehmes Tragen zu ermöglichen, in dem Prothesenschaft über ein Ventil aufblasbare Kissen anzuordnen. Dabei handelt es sich aber stets um den Stumpf ringsum umgebende, wenngleich zum Teil unterteilte, so doch zusammenhängende und damit einheitlich aufblasbare Gebilde. Gleiches gilt für das DE-GM 1 882 630, wonach zur Vermeidung dessen, daß der Stumpf auf dem Luftpolster schaukelt, innerhalb eines umlaufenden Luftkissens noch eine Einlage aus porösem plastischem Werkstoff vorgesehen ist.

Von daher liegt der Erfindung die Aufgabe zugrunde, eine Oberschenkelprothese nach Gattungsbegriff so auszubilden, daß sie es ermöglicht, ganz gezielt an vom Erfinder dafür als besonders geeignet ermittelten Stellen Volumenänderungen herbeiführen wie z.T. auch bewußt, im Sinne einer Korrekturpelotte, Druck ausüben zu können. Es hat sich nämlich gezeigt, daß bei Oberschenkelamputationen, um so mehr je kürzer der Stumpf ist, der Oberschenkelknochen (Femur) bestrebt ist, innerhalb des Stumpfes eine widernatürliche, sog. Abduktionsstellung (nach außen abgespreizte Stellung) einzunehmen, wodurch die für die Stabilisierung des Beckens erforderliche Muskelvorspannung auf der Außenseite des Stumpfes reduziert wird.

Die genannte Aufgabe ist nun durch die kennzeichnenden Merkmale des Anspruchs 1 gelöst. Die Unteransprüche geben darüberhinausgehend vorteilhafte Ausgestaltungsmöglichkeiten an.

Die betreffenden Backen an den nämlichen Stellen und mit der nämlichen Form erlauben es, den Änderungen des Stumpfvolumens auf möglichst verträgliche Weise Rechnung zu tragen, und dazu noch, die erwähnte Fehlstellung des Oberschenkelknochens nach Art einer Korrekturpelotte korrigieren zu können, indem dem Knochen durch die langgestreckte, im dorso-lateralen Bereich angeordnete Backe eine relative Adduktionsstellung (Anspreizstellung, d.h. eine zur Symmetrieebene des Körpers hin geschwenkt erscheinende Stellung) zu vermitteln ist. Weiterhin gestattet es die Erfindung sogar, insbesondere unter Hinzuziehung der Merkmale der Ansprüche 2 bis 5, in die Oberschenkelprothetik eine gewisse Konfektionierung einzuführen, indem sie auf verträgliche Weise verhältnismäßig weitgehende nachträgliche Anpassungen an individuelle Stümpfe ermöglicht. - Eine verhältnismäßig langgestreckte, mechanisch federbelastete Backe innerhalb eines Beinprothesenschaftes zeigt freilich prinzipiell bereits die GB-PS 269 606, während die DE-PS 742 945 eine mittels Schrauben verstellbare Backe angibt. -

Den Merkmalen der Ansprüche 2 bis 5 kommt auch insofern besondere Bedeutung zu, als sie es erlauben, unter Ausnutzung der nach Anspruch 1 erzielbaren guten Flächenhaftung des Schaftes am Stumpf einen aus orthopädischer wie auch gesamtmedizinischer Sicht äußerst wünschenswerten Stumpfendkontakt mit gezielt dosierbarer Stumpfendbelastung herzustellen. Dadurch läßt sich andererseits einer zu empfindlichen Einengung des Stumpfes im Umfangsbereich mit dadurch bedingten Stauerscheinungen vorbeugen und dazu noch ein wesentlicher Teil der Prothesenstützkraft in ganz natürlicher Weise über den Oberschenkelknochen in das Hüftgelenk einleiten.

Die Anordnung eines aufblasbaren Kissens im Stumpfendbereich neben einem ringförmigen im Umfangsbereich ist freilich aus der EP-OS 0 151 834 bei einer zusammenfaltbaren Unterschenkelprothese zum gelegentlichen Gebrauch z.B. in Duschkabinen prinzipiell bereits bekannt. Die betreffende Prothese weist aber keinen der Stumpfform auch nur halbwegs angepaßten Prothesenschaft auf.

Hinsichtlich der Ansprüche 7 bis 10 ist anzumerken, daß die betreffenden Maßnahmen für sich genommen teilweise bekannt sind, vor allem aus den die Fixierung einer Unterschenkelprothese mittels eines oder mehrerer aufblasbarer Wülste über dem Knie des Prothesenträgers betreffenden Veröffentlichungen US-PS 3 671 980 und AT-PS 364 073.

Nachfolgend werden einige Ausführungsbeispiele in Verbindung mit den Zeichnungen genauer beschrieben. Dabei zeigt
- Fig. 1: einen Längsschnitt durch den Schaft einer Oberschenkelprothese der beanspruchten Art mit Innenschaft, etwas schematisiert,
- Fig. 2: einen Querschnitt durch den gleichen Schaft und Innenschaft in der Ebene der Linie II-II von Fig. 1,
- Fig. 3: ein Detail etwa nach Fig. 1 in vergrößerter Darstellung mit zusätzlichen Einzelheiten,
- Fig. 4: schematisch das Prinzip einer anderen Ausführungsform,
- Fig. 5: ein entsprechendes Schema einer weiteren Ausführungsform und
- Fig. 6: ein Detail aus einem Längsschnitt durch eine Oberschenkelprothese ohne Innenschaft mit einer gänzlich anders ausgebildeten Backe.

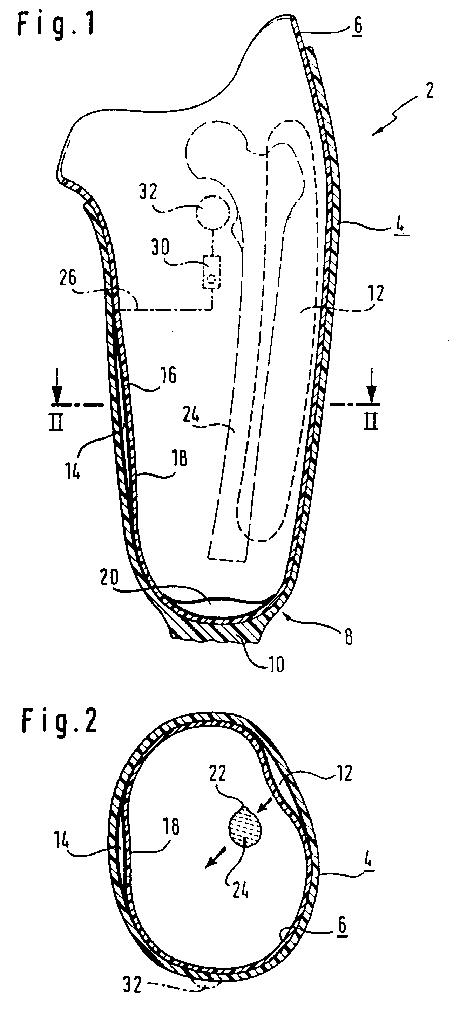

Die in Fig. 1 und 2 nur zum Teil dargestellte Oberschenkelprothese 2 weist in insoweit herkömmlicher Weise einen durch (nicht gezeigte) Armierungen oder dergl. verhältnismäßig steifen, schalenförmigen Außenschaft 4 und einen herausnehmbar darin eingesetzten, dem jeweiligen Stumpf individuell angepaßten, vergleichsweise flexiblen Innenschaft 6 auf. An das geschlossene distale Ende 8 des Außenschaftes 4 schließt sich ein zum Kniegelenk hin führendes säulenartiges Bauteil 10 an (Ummantelung in der Darstellung weggelassen).

Abweichend von herkömmlichen derartigen Prothesen sind nun im dargestellten Fall zwischen den längsverlaufenden Wandungen des Außenschaftes 4 und des Innenschaftes 6 zwei diskrete aufblähbare Kissen 12 und 14 angeordnet, die mit ihrer innenliegenden Wand wie z.B. 16 Backen bilden, von denen die Wandung 18 des Innenschaftes 6 in den betreffenden Bereichen nach innen gedrückt werden kann, um so eine lokale Reduzierung des Schaftvolumens zu erzielen. Ein weiteres solches Kissen, 20, befindet sich innenseitig im Innenschaft 6 an dessen distalem Ende, wo die Wandung 18 des Innenschaftes wegen der zweidimensionalen und zudem stärkeren Wölbung nicht gleichermaßen flexibel ist.

Genauer gesagt sind die Kissen 12 und 14 im dorso-lateralen Bereich im Anschluß an die Kante 22 des (gestrichelt angedeuteten) Oberschenkelknochens (Femur) 24 bzw. im medial-distalen Bereich angeordnet. Dabei weist das Kissen 12, sich von proximal nach distal erstreckend, eine langgestreckte, das Kissen 14 hingegen eine eher runde Form auf.

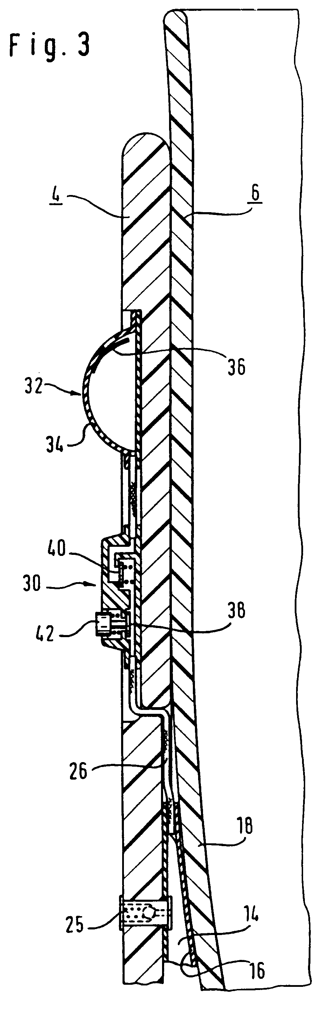

Wie durch die Pfeile in Fig. 2 angedeutet, erfährt der Oberschenkelknochen 24 durch das Kissen 12 in gewollter Weise eine mehr oder weniger starke Adduktion, womit, wie gesagt, die sonst sich bei Oberschenkelamputierten zumeist einstellende Abduktion korrigierbar ist. Daneben erlauben es die Kissen 12 und 14, das Schaftvolumen zu reduzieren und dem Stumpf im Schaft, genauer gesagt in dem Innenschaft 6, eine verstärkte Flächenhaftung zu vermitteln. Diese Flächenhaftung wiederum gestattet es, mit Hilfe des Kissens 20 einen Stumpfendkontakt mit dosierbarer Stumpfendbelastung herbeizuführen bzw. die Stumpfendbelastung in zumeist wünschenswerter Weise zu erhöhen. In diesem Zusammenhang kann gewünschtenfalls ein Druckbegrenzungsventil Anwendung finden, wie eines in Fig. 3 am Kissen 14 als Überdruckventil 25 gezeigt ist. Im übrigen aber gestatten es die Kissen, den Innenschaft 6 so voluminös zu gestalten, daß sich bei entlüfteten Kissen das Anziehen der Prothese ganz wesentlich erleichtert.

Wie in Fig. 1 angedeutet und genauer aus Fig. 3 ersichtlich, führt von dem jeweiligen Kissen, wie z.B. 14, ein feiner Schlauch 26 zu einem an der Prothese 2 angebrachten - oder aber auch separaten - Ventil 30, an das sich eine kleine manuell betätigbare Luftpumpe 32 anschließt, wie sie im Prinzip etwa von Parfumzerstäubern her bekannt ist. Im gezeigten Beispiel besteht die Luftpumpe 32 im wesentlichen aus einer sich durch Eigenspannung erweiternden flachen Gummibirne 34, an der ein klappenartiges Rückschlagventil 36 als Lufteinlaßventil angeordnet ist. Die Luftpumpe 32 ist unaufällig unter der Kleidung zu tragen und im Bedarfsfall durch diese hindurch durch wiederholtes Zusammendrücken und Entspannenlassen der Gummibirne 34 betätigbar. Mit ihr kann atmosphärische Luft, welche über das Ventil 36 angesaugt wird, durch das Ventil 30 hindurch in das Kissen 14 gedrückt werden, um dieses aufzublähen. Das Ventil 30 ist als Doppelrückschlagventil mit zwei Ventilgliedern 38 und 40 ausgestattet, deren eines, 38, von außen her über einen Druckknopf 42, der ebenfalls durch die Kleidung hindurch betätigbar ist, in seine Freigabestellung gebracht werden kann, um das Kissen 14 in die Atmosphäre zu entlüften. Gewünschtenfalls kann auch das vorausgehend erwähnte Druckbegrenzungsventil, wie etwa 25, in das Ventil 30 integriert sein.

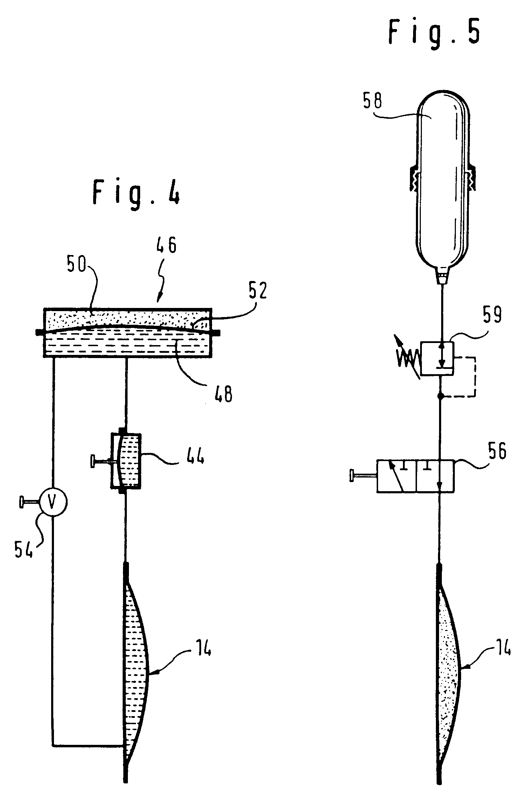

Fig. 4 zeigt rein schematisch eine alternative Ausführungsform, bei der Ventil 30 und Luftpumpe 32 aus den Figuren 1 und 3 durch eine Membranpumpe 44 in Verbindung mit einem Vorratsbehälter 46 ersetzt sind und aus dem Vorratsbehälter eine Flüssigkeit 48 in das Kissen, wie etwa 14, pumpbar ist. Zu diesem Zweck steht die Flüssigkeit 48 in dem Vorratsbehälter 46 unter dem Druck eines Gaspolsters 50, das durch eine Membran 52 von der Flüssigkeit 48 abgetrennt sein kann. Ein manuell betätigbares Rückflußventil 54 ermöglicht es, das Kissen 14 in den Vorratsbehälter 46 zurück zu entleeren. Der dazu erforderliche Druck auf das Kissen 14 kann vom Stumpf, etwa durch seitliches Ankippen der Prothese, aufgebracht werden. Anderenfalls ist es auch denkbar, zur Entleerung des Kissens eine zweite Membranpumpe oder dergl. vorzusehen. Im übrigen aber könnte der Vorratsbehälter 46 auch gleichfalls als Kissen ausgebildet werden, aus dem heraus durch manuellen Druck die Flüssigkeit 48 über ein manuell betägbares Absperrventil in das Kissen 14 verdrängt werden kann wie umgekehrt.

Wo, wie nach Fig. 1 und 2, innerhalb eines Prothesenschaftes mehrere Kissen, wie z.B. die Kissen 12, 14 und 20, vorgesehen sind, können diese selbstredend über entsprechend viele Ventile, wie z.B. 30 bzw. 54, mit einem einzigen Vorratsbehälter in Verbindung stehen.

An die Stelle manuell betätigbarer Pumpen, wie z.B. 32 und 44, können gewünschtenfalls natürlich auch elektrisch betätigbare, etwa aus einer mitgeführten Batterie speisbare Pumpen treten. Schließlich aber kann ein jedes der Kissen, wie z.B. 12, 14 und 20, gemäß Fig. 5 auch ohne Pumpen, nämlich durch ein manuell betätigbares Zweiwegeventil 56 hindurch mit einem gasförmigen Medium, vorzugsweise Luft, aus einem unter Druck stehenden Vorratsbehälter 58 heraus, aufgeblasen bzw. ins Freie entlüftet werden. Der Vorratsbehälter 58 ist dabei zweckmäßigerweise, wie gezeigt, als auswechselbare Patrone ausgebildet. Zwischen Vorratsbehälter 58 und Zweiwegeventil 56 ist ein Druckbegrenzungsventil in Gestalt eines Druckminderventils (Reduzierventils) 59 angeordnet.

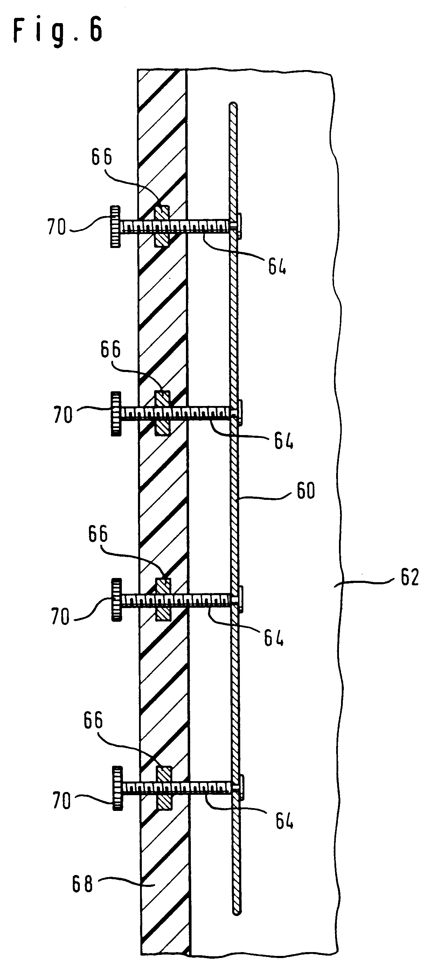

Fig. 6 gibt schließlich noch eine gänzlich andere Ausführungsform an, bei der die erfindungsgemäß vorgesehenen Backen als mechanisch gehaltene und verstellbare Backen ausgebildet sind. Genauer gesagt besteht eine solche Backe im gezeigten Fall aus einer Federblechplatte 60, die im Inneren des Schaftes 62 der Prothese, in diesem Falle eines einteiligen Schaftes, mittels mehrerer Schrauben 64 gehalten und abgestützt wird. Die Schrauben 64 sind in der Federblechplatte 60 drehbar verankert und durch Muttern 66 hindurchgeführt, die in die Wandung 68 des Schaftes 62 eingelassen sind. Zu ihrer individuellen Verstellung weisen die Schrauben 64 außerhalb der Wandung 68 Rändelköpfe 70 auf.

Eine solche Lösung wird naturgemäß am ehesten für die seitliche Wandung des Prothesenschaftes in Betracht kommen.

## Patentansprüche

1. Oberschenkelprothese mit schalenförmigem Schaft (4, 6) und einer Mehrzahl darin an der Schaftwandung (18) angeordneter, schaftein- und -auswärts verstellbarer Backen (16; 60), dadurch ***gekennzeichnet***, daß derartige Backen (16; 60) in länglicher Form im dorso-lateralen Bereich im Anschluß an die Kante (22) des Oberschenkelknochens (24), sich von proximal nach distal erstreckend, und, in annähernd runder Form, im medial-distalen Bereich angeordnet sind.

2. Oberschenkelprothese nach Anspruch 1, dadurch ***gekennzeichnet***, daß eine zusätzliche solche Backe im Stumpfendbereich angeordnet ist.

3. Oberschenkelprothese nach Anspruch 1 oder 2, dadurch ***gekennzeichnet***, daß zumindest eine der betreffenden Backen (60) mittels einer oder mehrerer Stellschrauben (64), vorzugsweise von der Außenseite des Schaftes (62) her, verstellbar ist.

4. Oberschenkelprothese nach einem der vorhergehenden Ansprüche, dadurch ***gekennzeichnet***, daß zumindest eine der betreffenden Backen von der innenliegenden Wand (z.B. 16) eines aufblähbaren Kissens (12, 14, 20) gebildet wird.

5. Oberschenkelprothese nach Anspruch 2 und 4, dadurch ***gekennzeichnet***, daß der Schaft (4, 6) im Stumpfendbereich im wesentlichen geschlossen.ist und die dortige Backe von einem auf der betreffenden Bodenpartie des Schaftes aufliegenden aufblähbaren Kissen (20) gebildet wird.

6. Oberschenkelprothese nach Anspruch 5, dadurch ***gekennzeichnet***, daß der Schaft in an sich bekannter Weise aus einem Außenschaft (4) und einem darin eingesetzten, den Konturen des Stumpfes genauer angepaßten Innenschaft (6) besteht und das betreffende, im Stumpfendbereich angeordnete Kissen (20) innerhalb des Innenschaftes (6) angeordnet ist.

7. Oberschenkelprothese nach Anspruch 6, dadurch ***gekennzeichnet***, daß der Innenschaft (6) zumindest in seinem Umfangsbereich flexibel ist und die gemäß Anspruch 1 dort auftretenden Backen von zwischen Außenschaft (4) und Innenschaft (6) angeordneten aufblähbaren Kissen (12, 14) gebildet werden.

8. Oberschenkelprothese nach einem der Ansprüche 4 bis 7, dadurch ***gekennzeichnet***, daß das Kissen (12, 14, 20) über ein Ventil (30; 56) aufblasbar bzw. aufpumpbar ist.

9. Oberschenkelprothese nach einem der Ansprüche 4 bis 7, dadurch ***gekennzeichnet***, daß das Kissen (12, 14, 20) mit einem Fluid (48) aus einem Vorratsbehälters (46; 58) befüllbar ist.

10. Oberschenkelprothese nach einem der Ansprüche 4 bis 9, dadurch ***gekennzeichnet***, daß das Kissen (12, 14, 20) mit einem Druckbegrenzungsventil, wie z.B. einem Überdruckventil (25) oder einem Druckminderventil (59), in Verbindung steht.

## Claims

1. Thigh prosthesis with a shell-type shaft (4, 6) and a plurality of cheeks (16; 60) arranged therein on the shaft wall (18) and adjustable inwards and outwards with respect to the shaft, characterized in that such cheeks (16; 60) are arranged in an elongate shape, in the dorsolateral region adjacent the edge (22) of the femur (24) and extending from proximal to distal and, in an approximately round shape, in the medial-distal region.

2. Thigh prosthesis according to Claim 1, characterized in that an additional such cheek is arranged in the stump end region.

3. Thigh prosthesis according to Claim 1 or 2, characterized in that at least one of the cheeks (60) in question can be adjusted by means of one or more adjusting screws (64), preferably from the outside of the shaft (62).

4. Thigh prosthesis according to one of the preceding claims, characterized in that at least one of the cheeks in question is formed by the interior wall (e.g. 16) of an inflatable cushion (12, 14, 20).

5. Thigh prosthesis according to the Claims 2 and 4, characterized in that the shaft (4, 6) is essentially closed in the stump end region, and the cheek there is formed by an inflatable cushion (20) lying on the respective bottom portion of the shaft.

6. Thigh prosthesis according to Claim 5, characterized in that the shaft consists, in a manner known per se, of an outer shaft (4) and of an inner shaft (6) fitted into the latter and adapted more exactly to the contours of the stump, and the cushion (20) in question which is arranged in the stump end region is arranged inside the inner shaft (6).

7. Thigh prosthesis according to Claim 6, characterized in that the inner shaft (6) is flexible, at least in its circumferential area, and the cheeks appearing there in accordance with Claim 1 are formed by inflatable cushions (12, 14) arranged between outer shaft (4) and inner shaft (6).

8. Thigh prosthesis according to one of Claims 4 to 7, characterized in that the cushion (12, 14, 20) can be inflated or pumped up via a valve (30; 56).

9. Thigh prosthesis according to one of Claims 4 to 7, characterized in that the cushion (12, 14, 20) can be filled with a fluid (48) from a storage container (46; 58).

10. Thigh prosthesis according to one of Claims 4 to 9, characterized in that the cushion (12, 14, 20) is in communication with a pressure-limiting valve such as, for example, a pressure relief valve (25) or a pressure-reducing valve (59).

## Revendications

1. Prothèse fémorale comportant une tige enveloppante (4, 6) et une pluralité de mâchoires (16 ; 60) ajustables vers l'intérieur et l'extérieur de ladite tige et disposées sur sa paroi intérieure (18),
caractérisée en ce que de telles mâchoires (16 ; 60), de forme allongée, sont disposées dans la région dorso-latérale en prolongation de l'arête (22) de l'os fémoral (24) et s'étendent de la région proximale vers la région distale, et, de forme sensiblement ronde, s'étendent dans la région médiane-distale.

2. Prothèse fémorale selon la revendication 1,
caractérisée en ce qu'une mâchoire supplémentaire est disposée dans la région terminale du moignon.

3. Prothèse fémorale selon la revendication 1 ou 2,
caractérisée en ce qu'au moins l'une desdites mâchoires (60) peut être ajustée au moyen d'une ou de plusieurs vis d'ajustage (64), de préférence à partir de la face extérieure de la tige (62).

4. Prothèse fémorale selon l'une des revendications précédentes,
caractérisée en ce qu'au moins l'une desdites mâchoires est formée par la paroi située à l'intérieur (par exemple 16) d'un coussin gonflable (12, 14, 20).

5. Prothèse fémorale selon les revendications 2 et 4,
caractérisée en ce que la tige (4, 6) est sensiblement fermée dans la région terminale du moignon, et que la mâchoire de cette région est formée par un coussin gonflable (20) reposant sur la portion de fond respective de la tige.

6. Prothèse fémorale selon la revendication 5,
caractérisée en ce que la tige consiste de manière connue en soi en une tige externe (4) et une tige interne (6) qui y est insérée et qui est adaptée de manière plus précise aux contours du moignon, et en ce que ledit coussin (20), disposé dans la région terminale du moignon, est placé à l'intérieur de la tige interne (6).

7. Prothèse fémorale selon la revendication 6,
caractérisée en ce que la tige interne (6) est souple au moins dans sa région périphérique, et que les mâchoires qui y sont prévues selon la revendication 1 sont formées par des coussins gonflables (12, 14) disposés entre la tige externe (4) et la tige interne (6).

8. Prothèse fémorale selon l'une des revendications 4 à 7,
caractérisée en ce que le coussin (12, 14, 20) peut être gonflé, éventuellement par une pompe, au travers d'une valve (30 ; 56).

9. Prothèse fémorale selon l'une des revendications 4 à 7,
caractérisée en ce que le coussin (12, 14, 20) peut être rempli d'un liquide (48) venant d'un réservoir (46; 58).

10. Prothèse fémorale selon l'une des revendications 4 à 9,
caractérisée en ce que le coussin (12, 14, 20) est en relation avec une soupape de limitation de la pression, telle que, par exemple, une soupape de surpression (25) ou une vanne de détente (59).
